# EUROPEAN PATENT SPECIFICATION

(11) **EP 0 563 037 B1**
(45) Date of publication and mention of the grant of the patent: **03.03.1999**
(21) Application number: 91902376.2
(22) Date of filing: 21.12.1990
(51) Int. Cl.: A61M 16/00

(54) **RESUSCITATION AID**
HILFSGERÄT FÜR DIE WIEDERBELEBUNG
DISPOSITIF DE REANIMATION

(43) Date of publication of application: 06.10.1993
(73) Proprietor: DON MICHAEL, T., Anthony, Bakersfield, CA 93305 (US)
(72) Inventor: DON MICHAEL, T., Anthony, Bakersfield, CA 93305 (US)
(74) Representative: Allman, Peter John
(86) International application number: US9007596
(87) International publication number: WO9211053

(56) References cited:
- WO-A-89/00418
- WO-A-89/11888
- FR-A- 1 374 116
- FR-A- 1 600 919
- GB-A- 2 198 958
- GB-A- 2 204 498
- US-A- 3 158 152
- US-A- 3 957 046
- US-A- 4 360 017
- US-A- 4 510 931
- US-A- 4 909 245

## Description

### BACKGROUND OF THE INVENTION

The present invention relates to resuscitation aids, particularly devices for facilitating mouth-to-mouth resuscitation.

As used herein, mouth-to-mouth resuscitation refers to methods in which air is forced from the lungs of a rescuer into the lungs of a victim of stopped breathing at regular intervals to provide the interchange of air necessary for respiration. If a victim of stopped breathing is to be saved from death, resuscitation must be started promptly after the cessation of breathing. At times, the heart may also have stopped, in which case simultaneous cardiac resuscitation will also be necessary.

Mouth-to-mouth resuscitation, frequently referred to as the "kiss of life", is a technique which is known to a significant portion of the population, particularly since it is not difficult to learn and does not require special equipment.

Unfortunately, the classic mouth-to-mouth technique requires direct contact between rescuer and victim, and many individuals find this aspect of the technique objectionable. Such objections have become even more prevalent because of the fear of transmission of the AIDS virus, given that a victim is often a stranger to a potential rescuer. Because of this fear, even trained paramedic personnel have become reluctant to administer mouth-to-mouth resuscitation.

Many devices have been developed for performing resuscitation in which no mouth-to-mouth contact is required between rescuer and victim. These devices usually involve inserting some type of tube into the airway of a victim. Among these devices are intubation devices, esophageal obturator airways and "bag valve mask" devices.

In order for such devices to be fully effective, they should establish an effective seal over the victim's mouth when air is being breathed into the victim. Many of the known devices are incapable of forming such a seal.

In addition, many known devices are relatively complicated and expensive so that they could not be made widely available for general use. Since resuscitation must be started within minutes after a stoppage of breathing, devices which cannot be made widely available and/or which can only be used by a small number of highly trained personnel are of little practical value.

Thus, mouth-to-mouth resuscitation remains the technique which offers the greatest hope of assistance to a victim of stopped breathing. Because the lips and associated facial muscles of such a victim are flaccid, virtually no known resuscitation aid can produce a perfect seal with the victim's lips. A nearly perfect seal can be created, however, if the rescuer purses his lips and then covers the victim's mouth. Such perfect seal is due in large measure to the ability of the rescuer to close his lips over the mouth area of the victim and thus perfectly conform to that area.

WO 89/11888 describes a disposable device which enables such a seal to be created while preventing the transmission of air and other fluids from the victim to the rescuer.

### SUMMARY OF THE INVENTION

It is a primary object of the present invention to provide a number of substantial improvements in the mask disclosed in the earlier application.

A more specific object of the invention is to provide an improved mask which can be more reliably positioned to establish an airway for a victim.

Another specific object of the invention is to provide an improved mask which is inexpensive to manufacture and highly portable, so that the mask can be easily carried by anyone and, because of its low cost, can be disposed of after use.

The above and other objects are achieved, according to the present invention, by a medical device for enabling a rescuer to administer mouth-to-mouth resuscitation to a victim while maintaining a sanitary barrier between victim and rescuer. comprising:
a sheet of flexible material forming a barrier to micro-organisms, said sheet being dimensioned to cover completely the victim's mouth, said sheet having an opening and a portion which surrounds said opening;
a tubular member defining a confined air passage extending through said opening and having a first portion located to he inserted into the victim's mouth and over the victim's tongue when said sheet is in place and a second portion located to be inserted into the mouth of the rescuer; and
means defining a one-way valve fastened to said tubular member for permitting free passage of air only from the rescuer to the victim characterised in that said portion of said sheet is frustoconical and circular in cross-section, the second portion of the tubular member is dimensioned so as to allow the rescuer's lips to contact the frustoconical portion thereby allowing them to form an air-tight seal with the victim's lips when the rescuer is blowing air into the victim's mouth, said sheet, including said frustoconical portion, being formed to permit the victim to exhale without obstruction when the rescuer's lips are withdrawn from the sheet.

### BRIEF DESCRIPTION OF THE DRAWING

Figure 1 is an elevational view of a preferred embodiment of a mask according to the present invention.
Figure 2 is a cross-sectional view of the airway portion of the mask of Figure 1, taken along the line II-II of Figure 1.
Figure 3 is a view similar to that of Figure 2 of a second embodiment of an airway according to the present invention.

### DESCRIPTION OF THE PREFERRED EMBODIMENTS

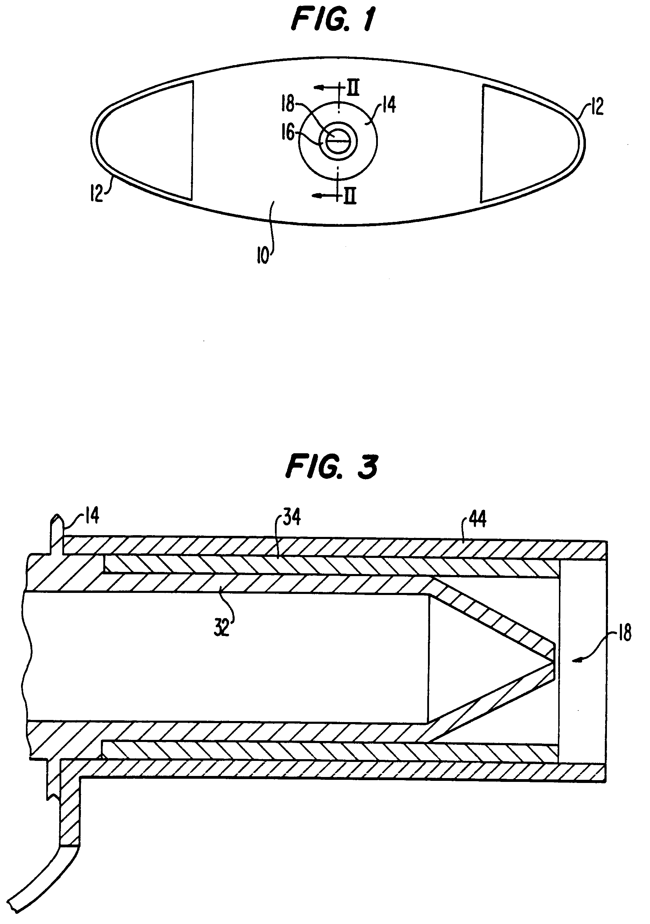

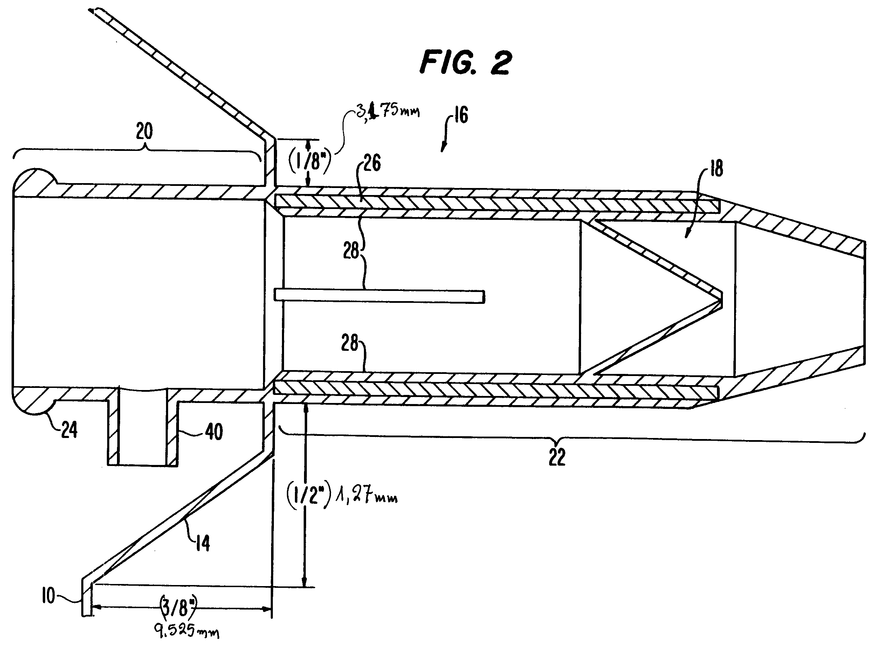

The mask shown in Figures 1 and 2 is essentially a one-piece molded plastic article preferably made of a flexible, transparent or semi-transparent plastic or a clear silicone rubber, the latter material presently being preferred. As shown in Figure 1, the mask is composed of a generally elliptical cover sheet 10 provided at its ends with straps 12 which are preferably molded integrally with sheet 10. Essentially at the center of sheet 10 there is provided a frustoconical lip portion 14 which is connected to a tube 16 provided at one end with a one-way valve 18 and constituting an airway via which air can be transferred from the rescuer to the victim. In addition, the mask is sized to cover the victim's nostrils. Even though the mask covers the victim's nostrils, it is still necessary for the rescuer to pinch the victim's nose when exhaling into the victim, in accordance with standard CPR practice. However, if the victim's nostrils are covered by the mask, the mask will serve as a barrier protecting the rescuer from contamination in the event the victim experiences vomiting through the nose during resuscitation.

As shown in Figure 2, tube 16 and its associated valve 18 are preferably molded integrally with sheet 10 and lip portion 14.

According to one advantageous feature of the present invention, the mask is to be secured in place on the victim, with straps 12 placed around the victim's ears, rather than being worn by the rescuer. Therefore, once tube 16 has been inserted into the victim's mouth and properly positioned, it will remain in place even if the rescuer should remove his mouth from tube 16.

Referring now more specifically to Figure 2, tube 16 includes a first portion 20 located at the side of the mask which will face the rescuer and a second portion 22 which will be inserted into the victim's mouth at the time the mask is placed on the victim. A bead 24 around the free end of portion 20 assists retention of portion 20 in the rescuer's mouth. Portion 22 is given a length sufficient to assure that it will rest upon the victim's tongue without extending so far as to contact the victim's throat, which would cause gagging, and possible vomiting. It is presently contemplated that this will be achieved if portion 22 has a length of the order of 50,8 to 57,2 mm (2 to 2 1/4 inches). Preferably, portion 20 has a length of the order of 19,1 to 25,4 mm (3/4 to 1 inch).

Lip portion 14 constitutes a significant component of the mask in that its configuration assures that the rescuer's lips can use lip portion 14 to form a "kiss" which creates an air-tight seal with the victim's lips during the times when the rescuer is blowing air into the victim's mouth, even though the victim's lips are usually flaccid. In effect, the form of lip portion 14 has been painstakingly developed to allow the rescuer's lips to establish an effective seal which makes possible efficient delivery of air to the victim's lungs to an extent comparable to that which can be achieved by direct contact mouth-to-mouth resuscitation.

Moreover, the function of lip portion 14 is such that when the rescuer's mouth is withdrawn, the victim can readily exhale around portion 14.

The preferred relevant dimensions for lip portion 14 are shown in Figure 2 for a mask made of 50 Durometer clear silicone rubber with lip portion 14 having a thickness of the order of 1,59 mm (1/16 inch). The dimensions shown in Figure 2 have been found to be suitable for individuals having a wide range of sizes. Bead 24 can assist the rescuer to place his mouth in the correct position relative to lip portion 14.

As can be seen from both Figures 1 and 2, valve 18 is preferably of the type having two flat sides which meet at a closing line, the valve thus being of the type which is referred to a "fish mouth" valve. When a valve of this configuration is made of the type of material described above, one effective example being as described above (50 Durometer clear silicone rubber, with a wall thickness of the order of 1,59 mm (1/16 inch)), it has been found that this valve will open easily to permit the passage of air in the direction from the rescuer to the victim, but will form a tight seal with respect to the transmission of air in the opposite direction. In fact, tests have shown that this valve is impermeable to the transmission of the AIDS virus in the direction from the victim to the rescuer.

At the free end of portion 22, tube 16 extends beyond valve 18 to provide a protective enclosure which will reduce the possibility that the victim's tongue can assume a position to block valve 18 or to interfere with its function.

Embedded within the region of portion 22 between lip portion 14 and valve 18 is a tube 26 which is relatively rigid, and which may be constituted, for example, by an acrylic or polycarbonate plastic having a thickness of the order of 1,59 mm (1/16 inch). Tube 26 is located to be interposed between the teeth of a victim when the mask is in place and thus serves to prevent the victim from involuntarily closing the airway or biting through tube 16 and from closing valve 18 with his tongue.

Tube 26 may be molded in place in the molded silicone rubber mask by being placed on a core having four radial projections which support the inner surface of tube 26, silicone rubber then being molded around tube 26 as the mask is being produced. When the resulting mask is removed from the mold, the radial projections of the core will leave slots 28, as shown in Figure 2. Between slots 28, silicone rubber adheres to the inner surface of tube 26.

According to a particularly advantageous embodiment of the tube 16 shown in Figure 2, this tube has an interior diameter of the order of 15,24 to 17,8 mm (0.6 to 0.7 inch) and a wall thickness of the order of 1 to 2,54 mm (0.04 to 0.1 inch), although other dimensions may prove suitable.

Generally, tube 16 has a circular cross section and it is particularly preferred that at least portion 20 have this cross section so that the rescuer can grip portion 20 in his mouth with his head having any orientation relative to that of the victim.

A second embodiment of a tube according to the present invention is illustrated in Figure 3. This tube is structurally simpler than that of Figure 2 and is composed of a molded body 32 of soft rubber having valve 18 at its distal end. Tube 32 is additionally provided in the vicinity of lip portion 14, with an annular shoulder 33 and carries a rigid plastic tube 34 which is positioned against shoulder 33. Tube 34 may be made of the same material as tube 26 of Figure 2 and performs essentially the same function. In addition, tube 34 extends over valve 18 to prevent the victim's tongue from interfering with the operation of valve 18. An embodiment employing the tube shown in Figure 3 is otherwise identical to that shown in Figure 2 and includes, in particular, a portion 22 which is to fit into the rescuer's mouth.

As noted earlier herein, the device is placed into use by inserting portion 22 into the victim's mouth so that the distal end of tube 16 or 32 lies upon the victim's tongue.

In the embodiments illustrated in Figures 2 and 3, valve 18 is protected from interference by the victim's tongue. However, if such protection is not provided, it is preferred that valve 18 be oriented relative to sheet 10 so that one of the flat, inclined surfaces of valve 18 will rest upon the victim's tongue and will act in the manner of a spatula to depress the tongue when air is being exhaled by the rescuer.

If, during a resuscitation procedure, the victim should experience vomiting, this can be dealt with by removing one of the straps 12 and pulling tube 16 out of the victim's mouth. Since the other strap 12 remains in position, the mask can be easily reinserted after the vomiting episode has terminated.

One feature of a mask according to the present invention is that it does not include any parts which obturate the victim's nose or which must be fit over the victim's chin, so that installation of the mask is simplified.

Embodiments of the invention could also be constructed to include an opening for connection of an auxiliary air supply device and/or to include electrodes via which impulses could be supplied to the victim's tongue and/or lips to perform a cardiac pacing or defibrillation function.

Thus, as shown in Figure 2, tube portion 20 may be optionally provided with a nipple 40 for connection to an auxiliary air or oxygen supply.

As shown in Figure 3, the mask may be packaged with a cylindrical metal electrode 44 connected to a wire for use, together with a second electrode, to effect defibrillation, or cardiac pacing. Electrode 44 may be open or closed at its distal end and can be used while mounted on tube 34 or by itself. When electrode 44 is open at its distal end, as shown in Figure 3, and is installed on tube 34, defibrillation or pacing can be performed simultaneously with resuscitation. The other electrode may be connected to any suitable location on the victim's body.

While the description above refers to particular embodiments of the present invention, it will be understood that many modifications may be made without departing from the spirit thereof. The accompanying claims are intended to cover such modifications as would fall within the true scope and spirit of the present invention.

The presently disclosed embodiments are therefore to be considered in all respects as illustrative and not restrictive, the scope of the invention being indicated by the appended claims, rather than the foregoing description.

## Claims

1. A medical device for enabling a rescuer to administer mouth-to-mouth resuscitation to a victim while maintaining a sanitary barrier between victim and rescuer, comprising:
a sheet of flexible material (10) forming a barrier to micro-organisms, said sheet (10) being dimensioned to cover completely the victim's mouth, said sheet (10) having an opening and a portion (14) which surrounds said opening;
a tubular member (16) defining a confined air passage extending through said opening and having a first portion (22) located to be inserted into the victim's mouth and over the victim's tongue when said sheet (10) is in place and a second portion (20) located to be inserted into the mouth of the rescuer; and
means (18) defining a one-way valve fastened to said tubular member (16) for permitting free passage of air only from the rescuer to the victim, said portion (14) being frustoconical, the second portion (20) of the tubular member (16) being dimensioned so as to allow the rescuer's lips to contact the frustoconical portion (14) thereby allowing them to form an air-tight seal with the victim's lips when the rescuer is blowing air into the victim's mouth, said sheet (10), including said frustoconical portion (14), being formed to permit the victim to exhale without obstruction when the rescuer's lips are withdrawn from the sheet (10).
characterised in that
said portion (14) of said sheet (10) is circular in cross-section.

2. A device as defined in claim 1 further comprising means surrounding said valve and serving as a barrier between said valve (18) and a victim's tongue when said first portion of said tubular member (16) is inserted into the mouth of the victim for preventing the victim's tongue from obstructing said valve (18).

3. A device as defined in claim 1 or 2 wherein said sheet (10), said tubular member (16) and said valve (18) are constituted by a one-piece moulded member.

4. A device as defined in claim 3 wherein said moulded member is of a silicone rubber.

5. A device as defined in any one of claims 1 to 4 further comprising a second tubular member (34) of a rigid material held in place relative to said first-recited tubular member (16) to surround the air passage, said second tubular member (34) serving as a bite block to prevent the victim from closing the air passage.

6. A device as defined in any preceding claim wherein said first portion (22) of said tubular member (16) is of a length to cause said valve (18) to be located above the tongue of the victim when said first portion (22) is inserted into the victim's mouth and to not induce vomiting by the victim.

7. A device as defined in any preceding claim wherein said means (18) defining a one-way valve are constituted by two planar members which contact one another along a line perpendicular to the air passage when said valve is closed, said planar members being moveable away from one another when the air pressure in said air passage is higher than that in the region exterior to and adjacent said valve.

8. A device as defined in any preceding claim wherein said sheet (10), said tubular member (16) and said one-way valve (18) are constituted by a one-piece moulded member.

9. A device as defined in claim 8 wherein said moulded member is made of a silicone rubber.

10. A device as defined in claim 9 wherein said valve is made of a 50 Durometer silicone rubber.

11. A device as defined in any preceding claim further comprising attachment means (12) attached to said sheet (10) for securing said device in place on the victim.

12. A device as defined in any preceding claim wherein said sheet (10) is dimensioned to cover the victim's nostrils.

## Patentansprüche

1. Medizinische Vorrichtung, um eine/n Retter/in dazu zu befähigen, eine Mund-zu-Mund-Beatmung auf ein Opfer anzuwenden während eine hygienische Barriere zwischen dem Opfer und dem/r Retter/in aufrechterhalten wird, die folgendes umfaßt:
eine dünne Lage eines elastischen Materials (10), die eine Barriere für Mikroorganismen bildet, wobei die dünne Lage (10) dazu abgemessen ist, den Mund des Opfers ganz zu überdecken, wobei die dünne Lage (10) eine Öffnung und einen Abschnitt (14) aufweist, der die Öffnung umgibt;
ein röhrenförmiges Glied (16), das einen beschränkten Luftdurchgang definiert, der sich durch die Öffnung erstreckt und einen ersten Abschnitt (22) aufweist, der dazu angeordnet ist, in den Mund des Opfers und über die Zunge des Opfers eingeführt zu werden, wenn sich die dünne Lage (10) an Ort und Stelle befindet, sowie einen zweiten Abschnitt (20), der dazu angeordnet ist, in den Mund des/r Retters/in eingeführt zu werden; und
Mittel (18), die ein in eine Richtung gehendes Ventil umfassen, das an dem röhrenförmigen Glied (16) befestigt ist, um einen freien Durchgang von Luft lediglich vom/von der Retter/in zum Opfer zu ermöglichen, wobei der Abschnitt (14) kegelstumpfförmig ist, wobei der zweite Abschnitt (20) des röhrenförmigen Glieds (16) so abgemessen ist, daß den Lippen des/der Retters/in ermöglicht wird, den kegelstumpfförmigen Abschnitt (14) zu berühren, wodurch ihnen ermöglicht wird, eine luftdichte Dichtung mit den Lippen des Opfers zu bilden, wenn der/die Retter/in Luft in den Mund des Opfers bläst, wobei die dünne Lage (10), die den kegelstumpfförmigen Abschnitt (14) umfaßt, dazu gebildet ist, dem Opfer zu ermöglichen, ohne Behinderung auszuatmen, wenn die Lippen des/r Retters/in von der dünnen Lage (10) zurückgezogen werden, dadurch gekennzeichnet, daß
der Abschnitt (14) der dünnen Lage (10) einen kreisförmigen Querschnitt aufweist.

2. Vorrichtung nach Anspruch 1, die ferner ein Mittel umfaßt, das das Ventil umgibt und als eine Barriere zwischen dem Ventil (18) und der Zunge eines Opfers dient, wenn der erste Abschnitt des röhrenförmigen Glieds (16) in den Mund des Opfers eingeführt wird, um zu verhindern, daß die Zunge des Opfers das Ventil (18) blockiert.

3. Vorrichtung nach Anspruch 1 oder 2, bei der die dünne Lage (10), das röhrenförmige Glied (16) und das Ventil (18) durch ein einstückiges Formglied gebildet werden.

4. Vorrichtung nach Anspruch 3, bei der das Formglied aus einem Silikongummi ist.

5. Vorrichtung nach einem der Ansprüche 1 bis 4, die ferner ein zweites röhrenförmiges Glied (34) aus einem starren Material umfaßt, das relativ zu dem erstgenannten röhrenförmigen Glied (16) so an Ort und Stelle gehalten wird, daß es den Luftdurchgang umgibt, wobei das zweite röhrenförmige Glied (34) als ein Beißblock dient, um zu verhindern, daß das Opfer den Luftdurchgang verschließt.

6. Vorrichtung nach einem der vorhergehenden Ansprüche, bei der der erste Abschnitt (22) des röhrenförmigen Glieds (16) eine solche Länge aufweist, daß das Ventil (18) dazu veranlaßt wird, sich oberhalb der Zunge des Opfers zu befinden, wenn der erste Abschnitt (22) in den Mund des Opfers eingeführt wird, und daß kein Erbrechen des Opfers induziert wird.

7. Vorrichtung nach einem der vorhergehenden Ansprüche, bei der die Mittel (18), die ein in eine Richtung gehendes Ventil definieren, durch zwei ebene Glieder gebildet werden, die einander entlang einer Linie berühren, die senkrecht zum Luftdurchgang liegt, wenn das Ventil geschlossen ist, wobei die ebenen Glieder voneinander wegbewegbar sind, wenn der Luftdruck im Luftdurchgang höher ist als der Luftdruck im Bereich außerhalb und angrenzend an das Ventil.

8. Vorrichtung nach einem der vorhergehenden Ansprüche, bei der die dünne Lage (10), das röhrenförmige Glied (16) und das in eine Richtung gehende Ventil (18) durch ein einstückiges Formglied gebildet werden.

9. Vorrichtung nach Anspruch 8, bei der das Formglied aus einem Silikongummi hergestellt ist.

10. Vorrichtung nach Anspruch 9, bei der das Ventil aus einem Silikongummi mit Durometerhärte 50 hergestellt ist.

11. Vorrichtung nach einem der vorhergehenden Ansprüche, die ferner ein Befestigungsmittel (12) umfaßt, das an der dünnen Lage (10) befestigt ist, um die Vorrichtung an dem Opfer an Ort und Stelle festzuhalten.

12. Vorrichtung nach einem der vorhergehenden Ansprüche, bei der die dünne Lage (10) dazu abgemessen ist, die Nasenlöcher des Opfers zu überdecken.

## Revendications

1. Dispositif médical pour permettre à un sauveteur d'administrer une réanimation bouche à bouche à une victime tout en maintenant une barrière sanitaire entre la victime et le sauveteur, comprenant:
une feuille de matière flexible (10) formant une barrière pour des micro-organismes, ladite feuille (10) étant dimensionnée pour recouvrir complètement la bouche de la victime, ladite feuille (10) possédant une ouverture et une portion (14) qui entoure ladite ouverture;
un élément tubulaire (16) définissant un passage confiné pour l'air s'étendant à travers ladite ouverture et possédant une première portion (22) disposée pour venir s'insérer dans la bouche de la victime et par-dessus la langue de la victime lorsque ladite feuille (10) est mise en place, et une seconde portion (20) disposée pour venir s'insérer dans la bouche du sauveteur; et
un moyen (18) définissant une soupape unidirectionnelle fixée audit élément tubulaire (16) pour permettre un passage libre d'air uniquement dans la direction du sauveteur vers la victime, ladite portion (14) étant de forme tronconique, la seconde portion (20) de l'élément tubulaire (16) étant dimensionnée pour permettre aux lèvres du sauveteur de venir se mettre en contact avec la portion tronconique (14), et de former ainsi un joint étanche à l'air avec les lèvres de la victime lorsque le sauveteur souffle de l'air dans la bouche de la victime, ladite feuille (10) englobant ladite portion tronconique (14) étant réalisée pour permettre à la victime d'expirer librement lorsque les lèvres du sauveteur se sont écartées de la feuille (10),
caractérisé en ce que
ladite portion (14) de ladite feuille (10) est de section transversale circulaire.

2. Dispositif tel que défini à la revendication 1, comprenant en outre un moyen entourant ladite soupape et faisant office de barrière entre ladite soupape (18) et la langue d'une victime lorsque ladite première portion dudit élément tubulaire (16) est insérée dans la bouche de la victime pour empêcher la langue de la victime d'obstruer ladite soupape (18).

3. Dispositif tel que défini à la revendication 1 ou 2, dans lequel ladite feuille (10), ledit élément tubulaire (16) et ladite soupape (18) sont constitués d'un élément moulé en une seule pièce.

4. Dispositif tel que défini à la revendication 3, dans lequel ledit élément moulé est réalisé en caoutchouc silicone.

5. Dispositif tel que défini à l'une quelconque des revendications 1 à 4, comprenant en outre un second élément tubulaire (34) réalisé en une matière rigide, maintenu en place par rapport audit élément tubulaire (16) cité en premier lieu, pour entourer le passage pour l'air, ledit second élément tubulaire (34) étant utilisé pour empêcher la victime d'obturer le passage pour l'air avec ses dents.

6. Dispositif tel que défini à l'une quelconque des revendications précédentes, dans lequel la longueur de ladite première portion (22) dudit élément tubulaire (16) est telle que ladite soupape (18) vient se disposer par-dessus la langue de la victime lorsque ladite première portion (22) est insérée dans la bouche de la victime et n'induit pas de vomissement de la part de la victime.

7. Dispositif tel que défini à l'une quelconque des revendications précédentes, dans lequel lesdits moyens (18) définissant une soupape unidirectionnelle sont constitués par deux éléments planaires qui viennent se mettre en contact l'un avec l'autre le long d'une ligne perpendiculaire au passage pour l'air lorsque ladite soupape est fermée, lesdits éléments planaires étant à même de s'écarter l'un de l'autre lorsque la pression d'air régnant dans ledit passage pour l'air est supérieure à celle régnant dans la région extérieure et adjacente à ladite soupape.

8. Dispositif tel que défini à l'une quelconque des revendications précédentes, dans lequel ladite feuille (10), ledit élément tubulaire (16) et ladite soupape unidirectionnelle (18) sont constitués d'un élément moulé en une seule pièce.

9. Dispositif tel que défini à la revendication 8, dans lequel ledit élément moulé est réalisé en caoutchouc silicone.

10. Dispositif tel que défini à la revendication 9, dans lequel ladite soupape est réalisée en un caoutchouc silicone de 50 duromètre.

11. Dispositif tel que défini à l'une quelconque des revendications précédentes, comprenant en outre un moyen de finition (12) fixé à ladite feuille (10) pour maintenir ledit dispositif en place sur la victime.

12. Dispositif tel que défini à l'une quelconque des revendications précédentes, dans lequel ladite feuille (10) est dimensionnée pour recouvrir les narines de la victime.
